# EUROPEAN PATENT APPLICATION

(11) **EP 4 809 961 A1**
(43) Date of publication of application: **23.09.2026**
(21) Application number: 26166133.4
(22) Date of filing: 19.03.2026
(51) Int. Cl.: A61B 90/14, A61B 90/11, A61B 34/30

(54) **FIDUCIAL FRAME**

(30) Priority: 21.03.2025 GB 202504136
(71) Applicant: Elekta Instrument AB, 103 93 Stockholm (SE)
(72) Inventor: THOMAS, Arn, 103 93 Stockholm (SE); ÅSTRÖM, Mattias, 103 93 Stockholm (SE)
(74) Representative: Haseltine Lake Kempner LLP

(57) **Abstract**

A fiducial frame comprising an attachment mechanism for attachment to a stereotactic frame. The fiducial frame further comprises fiducial imaging markers for aligning medical images to a frame of reference of the fiducial frame, and fiducial structures for coupling to a robotic surgical tool. Each fiducial structure comprises one of: a socket for receiving a convex locating structure of the robotic surgical tool; or a spherical protrusion for receiving a concave locating structure of the robotic surgical tool.

## Description

### Field

The present disclosure relates to a fiducial frame comprising an attachment mechanism for attachment to a stereotactic frame, to a method of manufacturing such a fiducial frame, to a surgical frame comprising such a fiducial frame attached to a stereotactic frame, and to a method of preparing a patient for surgery using such a surgical frame.

### Background

Robotic surgery is a growing field. In robotic surgery, a surgical robot performs the surgical procedure based on control instructions. Surgical robots are able to perform surgical procedures to a high degree of accuracy, and so are particularly useful for neurosurgery, in which there is limited room for error. Additionally, because surgical robots can be accurately controlled based on a scan of the treatment area, surgical procedures can be performed with minimal incisions.

In order to perform robotic surgery, it is important for the robot to know with a high degree of accuracy its position relative to the treatment area within the patient. Failure to correctly align the frame of reference of the robot to the frame of reference of the treatment area within the patient, could cause the surgical procedure to be performed in the incorrect place, and thereby cause surgical complications. In neurosurgery, the potential complications of incorrect alignment between the frame of reference of the robot and the frame of reference of the patient's brain may be particularly severe, causing damage to healthy brain tissue.

In order to correctly and accurately position a surgical robot relative to a treatment area, fiducial reference points can be used. Fiducial reference points, as defined herein, are points of reference having known fixed positions relative to the treatment area. Fiducial reference points are commonly provided by means of fiducial structures which are attached directly to the patient, for example directly to the patient's skull in the case of surgical neurosurgery. Such fiducial structures are known as bone fiducials. By imaging a patient's brain with the bone fiducials in place, the position of the bone fiducials relative to the treatment area within the brain can be calculated from the captured images. Therefore, such bone fiducials provide a means of positioning a surgical robot relative to the treatment area for surgery. In particular, by engaging a locating structure of the surgical robot with the bone fiducials, the robot is accurately aligned to the frame of reference of the bone fiducials; and by extension the location of the treatment area relative to the robot can be calculated (using the captured images).

A problem with bone fiducials, however, is that they require additional drilling to be performed through the skull, which is not desirable. Additionally, the accuracy and safety of the surgery is highly dependent on the accuracy of the calculated position of the bone fiducials relative to the treatment area. Accordingly, there is demand for a fiducial system which does not require the attachment of fiducial structures directly to a patient, and which provides reliable fixed fiducial reference points relative to which the position of the treatment area can be accurately and reliably determined.

### Summary

The subject matter of the present disclosure has been developed to address the problems described above.

In a first aspect, there is provided a fiducial frame comprising an attachment mechanism for attachment to a stereotactic frame, the fiducial frame further comprising:
fiducial imaging markers for aligning medical images to a frame of reference of the fiducial frame, and
fiducial structures for coupling to a robotic surgical tool, wherein each fiducial structure comprises one of:
   a socket for receiving a convex locating structure of the robotic surgical tool; or
   a spherical protrusion for receiving a concave locating structure of the robotic surgical tool.

Herein, a stereotactic frame is a frame which is configured to be secured to a patient's body, for example to a patient's head, in order to provide a stable reference frame (sometimes referred to herein as a stereotactic reference frame) which is fixed in position relative to the patient (and by extension relative to a treatment area in the patient). As will be described, a stereotactic frame according to the present disclosure may comprise attachment pins which press against the patient's body, for example press against the patient's head, in order to secure the stereotactic frame in place and prevent movement of the stereotactic frame relative to the patient.

Herein, the fiducial frame comprises one or more fiducial structures, wherein the one or more fiducial structures provide fiducial reference points for engagement by a surgical robot. When the fiducial frame is attached to a stereotactic frame, the fiducial structures thereby provide reference points, engageable by the surgical robot, which are fixed in position relative to the stereotactic frame and by extension to the treatment area. Accordingly, the fiducial frame enables a surgical robot to be reliably and stably located at a known position relative to the treatment area.

According to the first aspect, the fiducial frame comprises an attachment mechanism, such as a clamp, for attaching the fiducial frame to a stereotactic frame. The clamp may be configured to provide a secure connection which prevents movement of the fiducial frame relative to the stereotactic frame. Accordingly, by clamping the fiducial frame to the stereotactic frame (which itself can be securely attached to the body of the patient), the fiducial frame has a fixed position relative to the patient's body. In other words, the stereotactic frame defines a stereotactic frame of reference, which is fixed relative to the treatment area, and attaching the fiducial frame to the stereotactic frame thereby ensures that the fiducial structures are also fixed relative to the treatment area.

Accordingly, the fiducial frame according to the first aspect provides fiducial structures, which can be fixed in position relative to a patient's body (e.g. the patient's head) without needing to be drilled into the body of the patient. In fact, because medical imaging for the purpose of robotic surgery is commonly done with the use of a stereotactic frame to which a fiducial frame is attached, the fiducial frame according to the first aspect can be used with existing stereotactic frames in order to both to enable imaging of the patient's body, and to provide fiducial reference points for use by a surgical robot, without any additional attachments to the patient's body or other additional components being required.

Additionally, because the fiducial imaging markers and the fiducial structures are both part of the fiducial frame, the position of the fiducial structures relative to the fiducial imaging markers is known precisely, and is not changeable. By extension, the position of the fiducial structures relative to a frame of reference of images taken of the treatment area (which frame of reference may be defined by the imaging markers) does not need to be calculated. Therefore, the fiducial frame according to the first aspect ensures that the exact mapping between medical images taken of the patient's body (which images include the fiducial imaging markers) and the fiducial structures, is accurately known. In other words, the provision of both the fiducial imaging markers and the fiducial structures on the fiducial frame improves the accuracy with which the mapping from the frame of reference of the fiducial structures to the frame of reference of the treatment target is known.

In a second aspect, there is provided a surgical frame for use during robotic surgery, the surgical frame comprising:
a stereotactic frame comprising a plurality of fixation structures for securing the stereotactic frame to a body part (e.g. head) of a patient; and
a fiducial frame configured for attachment to the stereotactic frame, the fiducial frame comprising:
   fiducial imaging markers for aligning medical images to a frame of reference of the fiducial frame, and
   fiducial structures for coupling to a robotic surgical tool, wherein each fiducial structure comprises one of:
      a socket for receiving a convex locating structure of the robotic surgical tool; or
      a spherical protrusion for receiving a concave locating structure of the robotic surgical tool.

The surgical frame according to the second aspect may therefore be a system of parts, the system comprising the fiducial frame of the first aspect, and the stereotactic frame comprising the plurality of fixation structures. The stereotactic frame may comprise a first attachment surface on an outer surface thereof, and the fiducial frame may comprise a cooperating second attachment surface on an inner surface thereof. The stereotactic frame and the fiducial frame may engage one another at the first and second attachment surfaces, and may be secured to one another using a clamp.

Optional features of the first and second aspects will now be described.

Herein, where an "inward" direction or surface is referred to, it is to be understood that the direction or surface is generally towards or facing the head of a patient during use. Similarly, where an "outward" direction or surface is referred to, it is to be understood that the direction of surface is generally away from or facing away from the head of the patient during use. Similarly, an "inner" surface generally faces the patient's head during use, and an "outer" surface generally faces away from the patient's head during use.

The stereotactic frame may be for attachment to the head of the patient. The plurality of fixation structures may be configured for securing the stereotactic frame to the head of the patient.

The plurality of fixation structures may comprise inwardly extending attachment pins for engaging the body part (e.g. head) of the patient. The attachment pins may, for example, be adjustable and configured to press against the skull of the patient. Alternatively, the fixation structures may comprise openings, each opening configured to receive such an attachment pin.

The stereotactic frame may comprise a continuous rigid loop for arranging around the body part (e.g. head) of the patient. The continuous rigid loop may comprise the openings. Where the stereotactic frame comprises the attachment pins, the attachment pins may extend inwardly from the continuous loop. Where the stereotactic frame comprises a second attachment surface for engaging the fiducial frame, the second attachment surface may be provided on an outwardly facing surface of the continuous loop.

The fiducial frame may comprise at least one fiducial imaging panel. For example, the fiducial frame may comprise two fiducial imaging panels. The two imaging panels may be arranged on opposite sides of the frame from one another, so as to have the treatment area located therebetween in use. The two imaging panels may be oriented parallel to one another. They may be spaced apart from one another in the perpendicular direction.

Each fiducial imaging panel may be formed of an optically transparent and radiolucent material, for example plastic (such as polymethyl methacrylate "PMMA" or polycarbonate). Accordingly, the material of each fiducial imaging panel interferes minimally with x-ray or MRI imaging, and is also optically transparent for ease of positioning by a medical practitioner. However, each panel includes fiducial imaging markers which are not radiolucent (e.g. are radiopaque, and hence which are visible on x-ray and/or MRI scans) and which may not be optically transparent.

The fiducial imaging markers may be provided on, within, or through the at least one fiducial imaging panel. The fiducial structures may be attached to the at least one fiducial imaging panel. Each fiducial imaging panel may comprise at least one fiducial imaging marker, for example at least two fiducial imaging markers. Each fiducial imaging panel may comprise at least one fiducial structure, for example at least two fiducial structures.

Where the fiducial frame is a CT fiducial frame for use in CT scanning, the fiducial imaging markers may comprise radiopaque imaging markers which are visible to x-rays, for example copper strips. Where the fiducial frame is an MRI fiducial frame for use in MRI scanning, the fiducial imaging markers may comprise radiopaque imaging markers which are visible to MRI imaging, such as water-filled cavities within the fiducial imaging panel(s). Optionally, the cavities may be filled with a water-based copper sulphate (CuSO4) solution, to further improve their MRI visibility.

The fiducial structures may be attached to guide holes formed through the at least one fiducial imaging panel. For example, each fiducial structure may comprise a rivet which passes through a respective guide hole. A distal end of each rivet (e.g. the end which extends outwardly from the guide hole) may comprise the socket or protrusion.

The fiducial structures may be removeable from the at least one fiducial imaging panel. This may help with sterilising the fiducial panel prior to use and/or after use. The fiducial structures may, for example, be threaded for removeable attachment to the fiducial imaging panels.

Herein, where there are two (or more) fiducial imaging panels with a space therebetween, an outer surface of each fiducial imaging panel may be defined as the surface of the fiducial imaging panel which faces away from the adjacent fiducial imaging panel. Equivalently, an inner surface of each fiducial imaging panel may be defined as the surface of the fiducial imaging panel which faces towards the adjacent fiducial imaging panel.

The fiducial structures may extend outwardly from the fiducial frame, e.g. may extend from an outer surface of each fiducial imaging panel. The sockets or protrusions may therefore face outwardly from the fiducial imaging panels.

The fiducial structures may be aligned to one of an outer surface and an inner surface of the at least one fiducial imaging panel. In one example, the fiducial structures may be aligned to an outer surface of the at least one fiducial imaging panel. The fiducial imaging markers may similarly be aligned to the outer surface of the at least one fiducial imaging panel.

The fiducial imaging markers may form a two-dimensional shape which spans an area in the plane of the panel. The fiducial structures may be located within the area or adjacent to respective edges of the area.

In a third aspect there is provided a method of manufacturing a fiducial frame, for example a method of manufacturing a fiducial frame according to the first aspect.

The method of the third aspect may comprise:
providing at least one fiducial imaging panel;
forming at least one guide hole through the at least one fiducial imaging panel;
forming fiducial imaging markers on or through the at least one fiducial imaging panel, wherein the fiducial imaging markers are aligned relative to the guide holes;
attaching fiducial structures to the guide holes, wherein each fiducial structure comprises one of:
   a socket for receiving a convex locating structure of the robotic surgical tool; or
   a spherical protrusion for receiving a concave locating structure of the robotic surgical tool;
   the method further comprising:
providing a fiducial frame structure, the fiducial frame structure comprising an attachment mechanism for attachment to a stereotactic frame; and
attaching the at least one fiducial imaging panel to the fiducial frame structure.

Two fiducial panels may be provided and attached to the fiducial frame structure. At least two guide holes may be formed through the or each fiducial imaging panel. The fiducial imaging markers may be formed as a two-dimensional shape on the or each fiducial imaging panel. The two-dimensional shape may span an area. The at least two guide holes may be located within the area or adjacent to respective edges of the area.

The fiducial structures may be inserted into the guide holes from an outer surface or from an inner surface of the at least one panel. The fiducial structures may comprise rivets inserted into the guide holes from the outer surface or from the inner surface of the at least one panel.

In a fourth aspect there is provided a method of preparing a patient for robotic surgery using a surgical frame according to the second aspect or using a fiducial frame according to the first aspect.

The method according to the fourth aspect may comprise:
attaching a surgical frame according to the second aspect to the patient, such that the fiducial imaging markers are arranged adjacent to a treatment area of the patient; and
taking a medical image of the area to be treated, the image including the fiducial imaging markers.

The method may further comprise locating a surgical robot relative to the area to be treated by bringing a locating structure of the robotic surgical tool into contact with a fiducial structure of the fiducial structures.

The method may further comprise manoeuvring the surgical robot relative to the treatment area based on the medical image of the area to be treated.

Attaching the surgical frame to the patient's head may comprise first attaching a stereotactic frame to the patient's head, and then subsequently attaching a fiducial frame according to the first aspect to the stereotactic frame.

### Brief description of the drawings

The present disclosure will now be described in more detail, with reference to the accompanying drawings, in which:
Fig. 1 shows an example stereotactic frame according to the present disclosure;
Fig. 2a shows a first type of head fixation pin for use with the stereotactic frame of Fig. 1;
Fig. 2b shows a second type of head fixation pin for use with the stereotactic frame of Fig. 1;
Fig. 2c shows an attachment ring for use with a head fixation pin according to Fig. 2a or 2b;
Fig. 3 shows a fiducial frame according to the present disclosure, the fiducial frame attached to a stereotactic frame according to Fig. 1;
Fig. 4 shows a fiducial imaging panel from the fiducial frame of Fig. 3;
Fig. 5a shows a cross-sectional view of a fiducial structure according to a first example of the present disclosure;

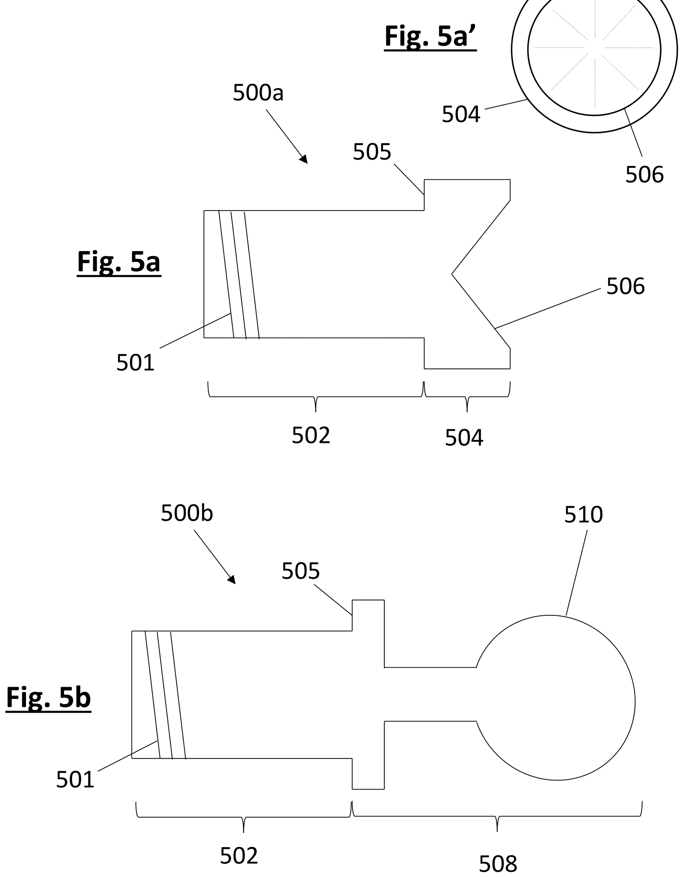

Fig. 5a' shows an end-on view of the fiducial structure of Fig. 5a;
Fig. 5b shows a cross-sectional view of a fiducial structure according to a second example of the present disclosure;

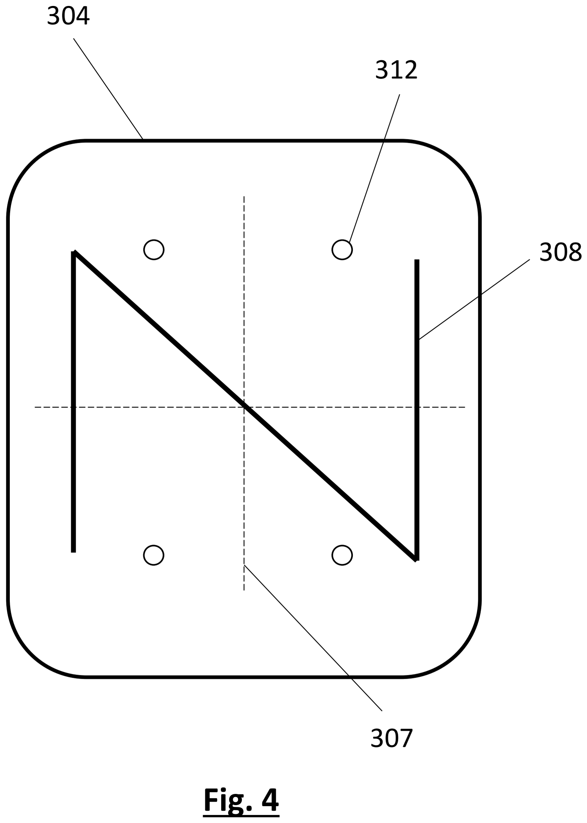

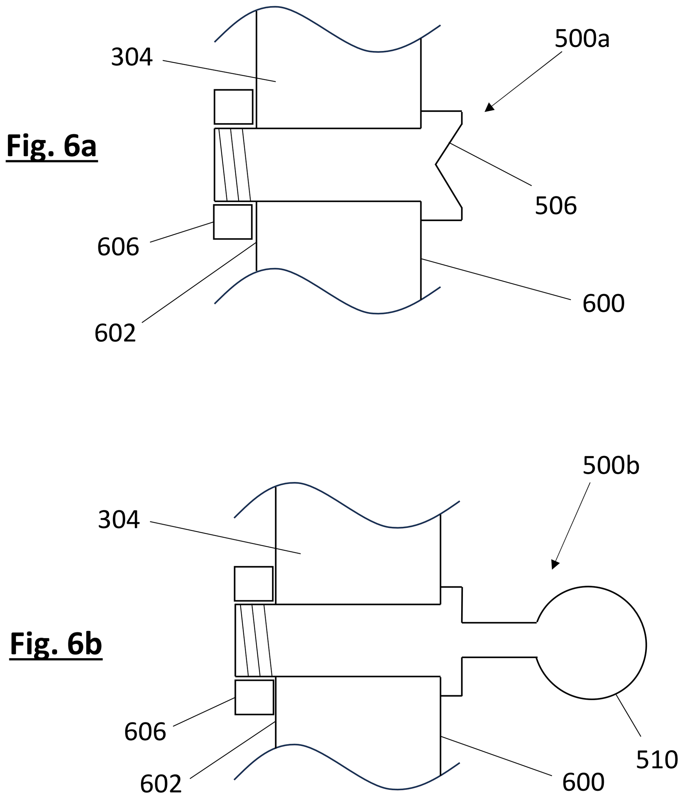

Fig. 6a shows a partial cross-sectional view of the fiducial imaging panel of Fig. 4, with a fiducial structure according to Fig. 5a attached thereto;
Fig. 6b shows a partial cross-sectional view of the fiducial imaging panel of Fig. 4, with a fiducial structure according to Fig. 5b attached thereto;

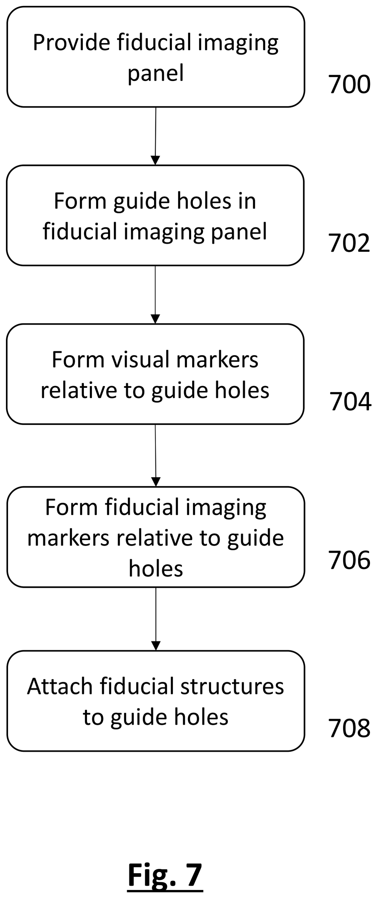

Fig. 7 is a flow chart for a method of manufacturing a fiducial imaging panel according to Fig. 4;

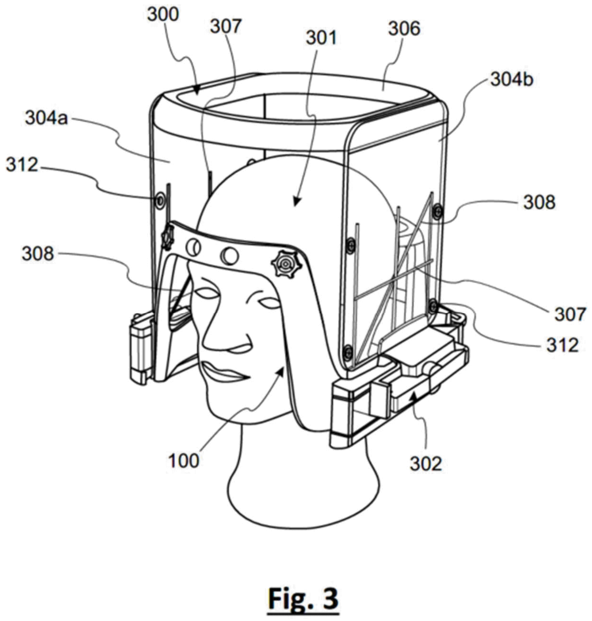

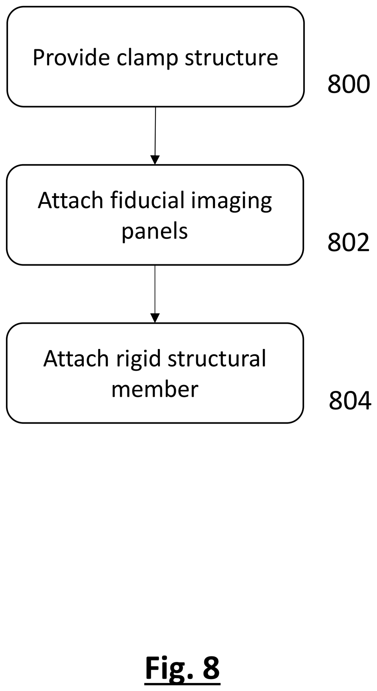

Fig. 8 is a flow chart for a method of manufacturing a fiducial frame according to Fig. 3; and

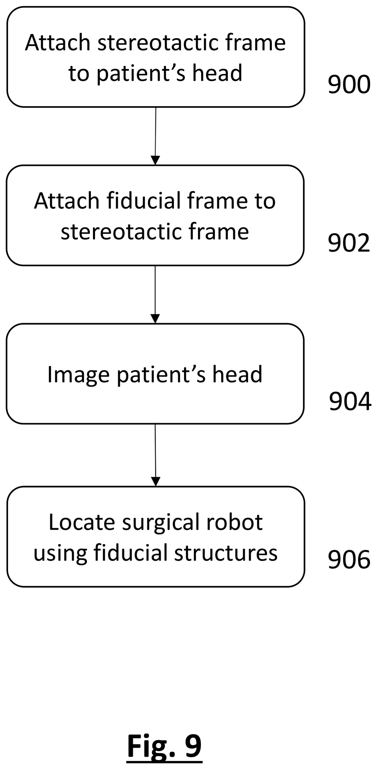

Fig. 9 is a flow chart for a method of preparing a patient for neurosurgery according to the present disclosure.

Like reference numerals are used for like components throughout the description.

### Detailed description

In the detailed description that follows, the present disclosure will be exemplified in the case in which the patient body part to be treated by robotic surgery is the patient's head. However, as the reader will understand, systems for use with other body parts are also within the scope of the present disclosure.

As described below, the system according to the present disclosure is usable for imaging a treatment area in the patient's brain prior to surgery, and is subsequently usable by a surgical robot for alignment to the treatment area in the patient's brain. As also described below, the system (surgical frame) includes a stereotactic frame which attaches to the patient's head to define a stable stereotactic reference frame, and a fiducial frame which attaches to the stereotactic frame thereby ensuring that fiducial structures associated with the fiducial frame are fixed in position relative to the stereotactic reference frame. The fiducial structures thereby provide stable reference points for aligning the surgical tool to the treatment area in the patient's brain.

Herein, various reference frames will be described. The first reference frame is the reference frame of the patient, and in particular of a treatment area within the patient (exemplified as a treatment area within the patient's brain tissue). The second reference frame is the reference frame of the stereotactic frame, which as described above can be fixed relative to the reference frame of the patient. The third reference frame is the reference frame of the fiducial frame, which is useable for imaging the treatment area, includes the fiducial structures, and can be fixed in place relative to the stereotactic frame (and by extension fixed in place relative to the reference frame of the patient). Finally, the fourth reference frame is the reference frame of the surgical robot. The reference frame of the surgical robot can be accurately positioned relative to that of the fiducial frame by engaging a locating structure of the surgical robot with fiducial structures on the fiducial frame. Therefore, by using the surgical frame of the present disclosure, and a known correspondence between images of the treatment area and locations of the fiducial structures, the reference frame of the surgical robot can be accurately positioned relative to that of the patient, thereby enabling surgical procedures to be accurately and reliably performed.

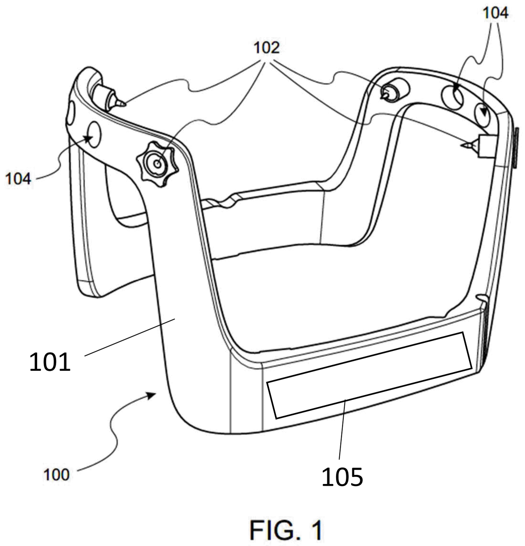

Fig. 1 shows an example stereotactic frame 100 according to the present disclosure. The stereotactic frame is configured for secure attachment to a patient's head. Once attached to the patient's head, the stereotactic frame cannot be moved relative to the patient's head. Accordingly, the stereotactic frame provides a stable reference frame which remains fixed in place relative to the patient's head, and by extension relative to the patient's brain tissue.

Before performing robotic surgery on a patient's brain, the patient's brain must first be imaged in order to determine where the regions of tissue to be treated are located. The stereotactic frame 100 as shown in Fig. 1 is therefore secured to the patient's head prior to imaging the patient's brain.

The stereotactic frame 100 comprises a rigid continuous loop 101. The rigid continuous loop 101 is sized to be arranged around a patient's head (as illustrated in Fig. 3). The outer surface of the rigid continuous loop 101 includes an attachment surface 105, which is configured to engage a corresponding attachment surface on a corresponding inner surface of a fiducial frame, for secure attachment to the fiducial frame by means of a clamp (which clamp is shown in Fig. 3).

The stereotactic frame 100 is secured in place by attachment pins 102 which are inserted into apertures 104 in the stereotactic frame 100, and which each include a distal tip which presses against the patient's skull in order to secure the stereotactic frame 100 in place and prevent movement of the stereotactic frame 100 relative to the patient's head. The attachment pins 102 extend inwardly from the rigid continuous loop 101.

In the depicted example of Fig. 1, four attachment pins 102 are used (two at the rear of the frame 100 and two at the front of the frame 100). However, in some examples, more than four pins 102, or fewer than four pins 102, may be used. Further, as shown in Fig. 1, more than four apertures 104 are present. In an exemplary example, eight apertures 104 may be present (four at the front of the frame 100, and four at the rear of the frame 100). Accordingly, the positions of the pins 102 can be selected according to the shape of the patient's head.

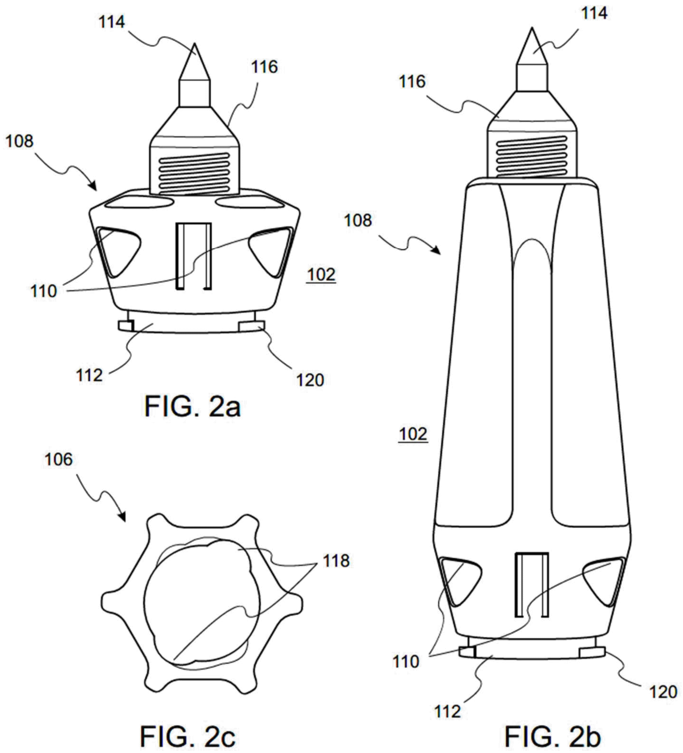

Fig. 2a shows a first example of an attachment pin 102. Fig. 2b shows a second example of an attachment pin 102. Fig. 2c shows an attachment ring 106 for use with an attachment pin 102 according to Fig. 2a or 2b.

Each attachment pin 102 includes an attachment portion 108 at a first end thereof. The attachment portion 108 includes attachment surfaces 110 for engaging an inner edge of a corresponding aperture 104 in the stereotactic frame 100. In order to initially secure an attachment pin 102 to the stereotactic frame 100, the attachment portion 108 is inserted into an aperture 104 such that the attachment surfaces 110 abut the inner surface of the stereotactic frame 100, and the attachment ring 106 is secured to the proximal end 112 of the attachment portion 108 such that the attachment ring 106 abuts the outer side of the stereotactic frame 100, thereby securing the attachment pin 102 to the stereotactic frame 100.

As shown, the distal end of each attachment pin 102 comprises a distal tip 114 (e.g. a pointed metallic tip 114) for engaging a patient's skull. With the attachment pin 102 attached to the stereotactic frame 100 as described above, the distal tip 114 points inwards towards the patient's skull. The distal tip 114 is mounted to an intermediate portion 116. The intermediate portion is threaded relative to the attachment portion 108, such that the extent of projection of the distal tip 114 from the attachment portion 108 can be increased or decreased by rotating the intermediate portion 116 relative to the attachment portion 108. This enables the position of the distal tip 114 to be adjusted in order to press against the patient's skull with sufficient force to secure the stereotactic frame 100 in place (for example, with a force of at least 500N for most adult patients, or a lower force where the patient is a child or the patient's skull is otherwise weakened). Further, as shown in the figures, the attachment pin 102 of Fig. 2b has a longer attachment portion 108 than that of the attachment pin 102 of Fig. 2a. Accordingly, the attachment pin 102 of Fig. 2b may be better suited for securing the stereotactic frame 100 to the head of a child.

In the present disclosure, a proximal direction is defined as a direction that is towards a stereotactic frame 100 (e.g. radially outwards and towards a surface of a stereotactic frame, or radially inwards and towards a surface of a stereotactic frame 100). Further, a distal direction is defined as a direction that is away from a stereotactic frame (e.g. either radially inwards and away from a surface of a stereotactic frame, or radially outwards and away from a surface of a stereotactic frame).

As shown in Fig. 2c, the attachment ring 106 comprises a female bayonet attachment 118 configured to engage a corresponding male bayonet attachment 120 at the proximal end of the attachment portion 108. Accordingly, the attachment ring 106 is attached to the attachment portion 108 by rotating the attachment ring 106 relative to the attachment portion 108.

Once the stereotactic frame 100 has been secured to the patient's head using attachment pins 102 as described above, a fiducial frame can be attached to it, by clamping the fiducial frame to the attachment surface 105 using a clamp 302.

Fig. 3 shows a fiducial frame 300 according to the present disclosure, the fiducial frame 300 being attached to the stereotactic frame 100 of Fig. 1 by means of a clamp 302. The fiducial frame includes a first fiducial imaging panel 304a on a first side thereof, and a second fiducial imaging panel 304b on a second side thereof. The fiducial imaging panels 304a, 304b are parallel to one another, and are spaced from one another to provide a space therebetween for a patient's head 301. They are configured to be provided at respective lateral sides of the patient's head 301 (e.g. a left-side and a right-side of the patient's head). A rigid structural member 306 is provided at a first (e.g. upper) end of the fiducial frame 300. Each of the fiducial panels 304a, 304b is attached at the first end thereof to the rigid structural member 306, and at the second (e.g. lower) end thereof to the clamp 302. The provision of the rigid structural member 306 ensures that the panels 304a, 304b do not move relative to one another or relative to the stereotactic frame. The panels 304a, 304b may be substantially identical to one another.

Fig. 4 shows a fiducial imaging panel 304 according to the present disclosure, for use as the first fiducial imaging panel 304a and/or as the second fiducial imaging panel 304b. The fiducial imaging panel 304 is rectangular in shape, optionally with rounded corners, and is formed of transparent plastic, such as transparent PMMA or transparent polycarbonate. Accordingly, it is substantially optically transparent, and is also substantially radiolucent (more specifically, transparent to x-rays, and/or transparent to MRI imaging).

As shown in Fig. 4, four guide holes 312 are formed through the fiducial imaging panel 304, and are arranged in the fiducial imaging panel 304 to define four corners of a square or rectangle. However, as the reader will understand, more than four guide holes 312, or fewer than four guide holes 312, may be formed through the imaging panel 304. Also provided on an outer surface of the fiducial imaging panel 304 are visual markers 307, which are optically opaque and are provided in the form of a cross; and fiducial imaging markers 308, which form a z-shape on the imaging panel.

The visual markers 307 may be radiolucent (i.e. not visible to x-rays and/or to MRI imaging). Where the fiducial frame 300 of Fig. 3 is to be used in CT imaging, the fiducial imaging markers 308 are formed of copper strips, which are visible to x-rays and therefore will show up in CT images taken of the patient's brain tissue. Where the fiducial frame 300 of Fig. 3 is to be used in MRI imaging, the fiducial imaging markers 308 comprise cavities within the fiducial imaging panel 304 which are filled with water or with water-based copper sulphate (CuSO4) solution, both of which are visible to x-rays and therefore will show up in MRI images taken of the patient's brain tissue.

In some examples, the visual markers 307 may be dispensed with.

As will be described in more detail in Fig. 7, the visual markers 307 and the fiducial imaging markers 308 are aligned relative to the guide holes 312. Therefore, the location of the guide holes 312 relative to the visual markers 307 and relative to the fiducial imaging markers 308 is precisely known.

By using the fiducial frame 300 according to Fig. 3, which includes imaging panels 304 according to Fig. 4, the visual markers 307 provide for easy locating of the fiducial frame 300 relative to the patient's head. Furthermore, the fiducial imaging markers 308 enable the frame of reference of the fiducial frame 300 to be identified in scan images of the treatment area which are obtained when the fiducial frame 300 is in place. Therefore, it is possible using the fiducial frame 300 to accurately determine the mapping between the frame of reference of the treatment area and the frame of reference of the fiducial frame. This mapping therefore enables the surgical treatment to be accurately planned, in the knowledge that the mapping between the fiducial frame of reference and the treatment area frame of reference is accurately known.

Fig. 5a shows a cross-sectional view of a fiducial structure 500a of a first type for inserting into the guide holes 312 of Figs. 3-4. As shown, the fiducial structure 500a of the first type includes a proximal rivet portion 502, which is sized to be inserted into a guide hole 312; and a distal socket portion 504, which includes a concave conical socket profile 506 for receiving a convex locating structure of a surgical robot (not shown). The conical socket comprises a cone having a circular base, such that the conical socket provides a circular opening when the fiducial structure 500a is viewed end-on, in order to be able to receive a spherical protrusion therein. This is illustrated in Fig. 5a'. The fiducial structure 500a is formed of metal, for example aluminium. On an outer surface of the proximal rivet portion 502 there is provided an external thread 501, so that the fiducial structure 500a can be removably attached to the fiducial imaging panel 304.

Fig. 5b shows a cross-sectional view of a fiducial structure 500b of a second type for inserting into the guide holes 312 of Figs. 3-4. As shown, the fiducial structure 500b of the second type includes a proximal rivet portion 502, which is sized to be inserted into a guide hole 312; and a distal protrusion portion 508, which includes a spherical protrusion 510 for receiving a concave locating structure of a surgical robot (such as a conical socket having a circular opening, not shown). The fiducial structure 500b is formed of metal, for example aluminium. On an outer surface of the proximal rivet portion 502 there is provided an external thread 501, so that the fiducial structure 500b can be removably attached to the fiducial imaging panel 304.

Each of the fiducial structure 500a of the first type and the fiducial structure 500b of the second type includes a proximally-facing lip 505, located at the interface between the proximal portion and the distal portion. The lip 505 is configured in use to engage an outer surface of the fiducial imaging panel 304 to which the fiducial structure is attached. Accordingly, the proximal-facing lip 505 extends to an outer diameter which is larger than an outer diameter of the proximal rivet portion 502.

The four guide holes 312 of the panel 304 shown in Fig. 3 can either be fitted with fiducial structures 500a of the first type, so as to provide four sockets on the panel 304 for receiving convex locating structures of a robotic surgical tool; or the four guide holes 312 of the panel 304 shown in Fig. 3 can be fitted with fiducial structures 500b of the second type, so as to provide four protrusions on the panel 304 for receiving concave locating structures of a robotic surgical tool. That is to say, the fiducial frame 300 of Fig. 3 will either be fitted with fiducial structures 500a of the first type, or with fiducial structures 500b of the second type. Therefore, in the case as illustrated in Figs. 3-4 in which there are four guide holes in each panel 304, each panel will include four fiducial structures 500a of the first type, or four fiducial structures 500b of the second type.

Fig. 6a illustrates a partial cross-sectional view of a panel 304 with fiducial structures 500a of the first type fitted. Labelled on Fig. 6a is an outer surface 600 of the panel 304 (which faces away from the other panel 304 of the fiducial frame 300); and an inner surface 602 of the panel (which faces towards the other panel 304 of the fiducial frame 300). The proximal-facing surface 505 of the fiducial structure 500a abuts the outer surface 600 of the fiducial imaging panel 304. A nut 606 is threaded onto the thread 501 at the inner surface 602 of the fiducial imaging panel 304, so as to secure the fiducial structure 500a in place.

Fig. 6b illustrates a partial cross-sectional view of panel 304 with fiducial structures 500b of the second type fitted. Labelled on Fig. 6b is an outer surface 600 of the panel 304 (which faces away from the other panel 304 of the fiducial frame 300); and an inner surface 602 of the panel (which faces towards the other panel 304 of the fiducial frame 300). The proximal-facing surface 505 of the fiducial structure 500b abuts the outer surface 600 of the fiducial imaging panel 304. A nut 606 is threaded onto the thread 501 at the inner surface 602 of the fiducial imaging panel 304, so as to secure the fiducial structure 500a in place.

Because the fiducial structures 500a/500b are inserted into the guide holes 312, relative to which the fiducial imaging markers 308 are aligned, the location of the fiducial structures relative to the fiducial imaging markers 308 is precisely known. Accordingly, by using the fiducial imaging markers 308 to identify and locate the frame of reference of the fiducial frame in the scan images of the treatment area, the location of the fiducial structures relative to specific treatment locations within the treatment area is, by extension, precisely known.

A method of manufacturing a fiducial imaging panel 304 according to Fig. 4 will now be described, with reference to the flow chart of Fig. 7.

In step 700, a precursor to the fiducial imaging panel 304 is provided. In its initial state (prior to the processing at steps 702-708), the panel is provided as a plain, featureless, rectangular plastic panel.

In step 702, guide holes 312 are formed through the fiducial imaging panel. They are formed using a jig, which accurately positions the guide holes at predefined locations on the fiducial imaging panel and in a predefined arrangement. The guide holes may be drilled, stamped, or laser etched through the plastic panel.

In step 704, visual markers 307 are formed on a surface of the panel. The surface on which the visual markers are formed thereafter becomes the outer surface of the panel (i.e. the surface of the panel which will face outwardly in the assembled fiducial frame). The visual markers 307 are positioned on the panel using the guide holes 312 as reference points. Therefore, the visual markers 307 have a precisely known location on the panel relative to the guide holes 312. The visual markers 307 may be printed onto the panel, drawn onto the panel, or transferred onto the panel, using the guide holes to correctly and accurately position the visual markers 307.

In step 706, fiducial imaging markers 308 are then formed on the surface of the panel or within the panel (as appropriate).

In the case of a panel for use in CT scanning, in which the fiducial imaging markers are copper strips, the copper strips are attached to the external surface of the panel (i.e. on the same surface as the visual markers 307). The fiducial imaging markers 308 are positioned on the panel using the guide holes 312 as reference points. Therefore, the fiducial imaging markers 308 have a precisely known location on the panel relative to the guide holes 312.

In the case of a panel for use in MRI imaging, in which the fiducial imaging markers are water-filled cavities or copper sulphate solution-filled cavities, channels are formed through the panel by drilling, machining or laser cutting; and pre-filled capsules of water or copper sulphate solution are secured within the channels to provide the fiducial imaging markers 307. Alternatively, two thin plates may be glued together with channels formed therebetween and filled with water or copper sulphate solution. In either case, the channels are positioned within the panel using the guide holes 312 as reference points.

In step 708, a fiducial structure 500a/500b is inserted into each guide hole 312, such that the proximal rivet portion extends into the guide hole 312, and such that the proximally-facing lip 505 abuts the outer surface of the panel. Each fiducial structure 500a/500b is then secured in place, using a nut 606 or other means as appropriate.

Accordingly, a panel 304 according to Fig. 4 is produced. Because the guide holes 312 are used for positioning each of the visual markers 307, the fiducial imaging markers 308, and the fiducial structures 500a/500b, the positions of the fiducial structures 500a/500b relative to the visual markers 307 and the fiducial imaging markers 308 is precisely known.

A method of manufacturing a fiducial frame 300 according to Fig. 3 will now be described, with reference to the flow chart of Fig. 8.

At step 800, a clamp structure for the fiducial frame is provided, the clamp structure including clamp 302.

At step 802, two fiducial imaging panels 304 (e.g. manufactured according to the method of Fig. 7) are attached to the clamp structure, so that they are arranged parallel to one another, on opposing sides of the clamp structure from one another.

At step 804, a rigid structural member 306 is attached to the two fiducial imaging panels 304, at an opposite end of the panels from the clamp structure.

A method of preparing a patient for robotic surgery, using a fiducial frame 300 according to Fig. 3 will now be described, with reference to the flow chart of Fig. 9.

At step 900, the stereotactic frame 100 is attached to the patient's head. This is done by adjusting the attachment pins 102 such that they press against the patient's head, thus securing the stereotactic frame in place.

At step 902, the fiducial frame 300 is attached to the stereotactic frame 100. In particular, the fiducial frame 300 is attached to the attachment surface 105 of the stereotactic frame 100 using the clamp 302.

At step 904, the patient's head is imaged, either using a CT scanner, or using an MRI scanner.

At step 906, following the imaging, a surgical robot is located relative to the fiducial frame 300, by means of the fiducial structures 312. In particular, a locating structure of the surgical robot is brought into contact with the fiducial structures 312, such that the position of the surgical robot relative to the fiducial structures 312 is precisely known.

Having performed steps 900-906, the surgical robot can then be used to performed surgery on the patient, safe in the knowledge that position of the frame of reference of the surgical robot is precisely known relative to the frame of reference of the treatment area in the patient.

It is to be understood that the above description is intended to be illustrative, and not restrictive. Many other implementations will be apparent to those of skill in the art upon reading and understanding the above description. Although the present disclosure has been described with reference to a specific example implementation, it will be recognized that the disclosure is not limited to the implementations described, but can be practiced with modification and alteration insofar as such modification(s) and alteration(s) remain within the scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A fiducial frame comprising an attachment mechanism for attachment to a stereotactic frame, the fiducial frame further comprising:
fiducial imaging markers for aligning medical images to a frame of reference of the fiducial frame, and
fiducial structures for coupling to a robotic surgical tool, wherein each fiducial structure comprises one of:
a socket for receiving a convex locating structure of the robotic surgical tool; or
a spherical protrusion for receiving a concave locating structure of the robotic surgical tool;
wherein the fiducial frame comprises two fiducial imaging panels, wherein fiducial imaging markers are provided on, within, or through each fiducial imaging panel, and wherein fiducial structures are attached to each fiducial imaging panel; and
wherein the two fiducial imaging panels are oriented parallel to one another and are located on opposite sides of the fiducial frame from one another.

2. A surgical frame for use during robotic surgery, the surgical frame comprising:
a stereotactic frame comprising a plurality of fixation structures for securing the stereotactic frame to a body part of a patient; and
a fiducial frame configured for attachment to the stereotactic frame, the fiducial frame comprising:
fiducial imaging markers for aligning medical images to a frame of reference of the fiducial frame, and
fiducial structures for coupling to a robotic surgical tool, wherein each fiducial structure comprises one of:
a socket for receiving a convex locating structure of the robotic surgical tool; or
a spherical protrusion for receiving a concave locating structure of the robotic surgical tool;
wherein the fiducial frame comprises two fiducial imaging panels, wherein fiducial imaging markers are provided on, within, or through each fiducial imaging panel, and wherein fiducial structures are attached to each fiducial imaging panel; and
wherein the two fiducial imaging panels are oriented parallel to one another and are located on opposite sides of the fiducial frame from one another.

3. The frame according to claim 2, wherein the stereotactic frame is for attachment to the head of the patient, and wherein the plurality of fixation structures are for securing the stereotactic frame to the head of the patient.

4. The frame according to claim 2 or claim 3, wherein the plurality of fixation structures comprise inwardly extending attachment pins for engaging the body part of the patient, or openings for receiving such attachment pins.

5. The frame of any preceding claim, wherein the fiducial structures are attached to guide holes formed through the at least one fiducial imaging panel.

6. The frame of claim 5, wherein the fiducial structures are removeable from the at least one fiducial imaging panel.

7. The frame according to any preceding claim, wherein the fiducial structures are aligned to one of an outer surface and an inner surface of the at least one fiducial imaging panel.

8. The frame according to any preceding claim, wherein the fiducial imaging markers form a two-dimensional shape covering an area in the plane of the panel, and wherein at least two fiducial structures are located within the area or adjacent to respective edges of the area.

9. The frame according to any preceding claim, wherein the fiducial structures face outwardly from the frame.

10. The frame according to any preceding claim, wherein each fiducial structure comprises a conical socket for receiving a convex locating structure of the robotic surgical tool.

11. A method of manufacturing a fiducial frame, the method comprising:
providing two fiducial imaging panels;
forming at least one guide hole through each fiducial imaging panel;
forming fiducial imaging markers on or through each fiducial imaging panel, wherein the fiducial imaging markers are aligned relative to the guide holes;
attaching fiducial structures to the guide holes, wherein each fiducial structure comprises one of:
a socket for receiving a convex locating structure of the robotic surgical tool; or
a spherical protrusion for receiving a concave locating structure of the robotic surgical tool;
the method further comprising:
providing a fiducial frame structure, the fiducial frame structure comprising an attachment mechanism for attachment to a stereotactic frame;
attaching the fiducial imaging panels to the fiducial frame structure so that they are oriented parallel to one another and are located on opposite sides of the fiducial frame structure from one another.

12. The method of claim 11, wherein at least two guide holes are formed through the at least one fiducial imaging panel, wherein the fiducial imaging markers are formed as a two-dimensional shape on the at least one panel, wherein the two-dimensional shape spans an area, and wherein the at least two guide holes are located within the area or adjacent to respective edges of the area.

13. The method of claim 11 or claim 12, wherein the fiducial structures are inserted into the guide holes from an outer surface or from an inner surface of the at least one panel.

14. A method of preparing a patient for robotic surgery, the method comprising:
attaching a surgical frame according to claim 2 to the patient, such that the fiducial imaging markers are arranged adjacent to a treatment area of the patient; and
taking a medical image of the area to be treated, the image including the fiducial imaging markers.
